(19) Europäisches Patentamt
European Patent Office
Office européen des brevets

(11) Publication number: **0 385 172 A1**

(12) **EUROPEAN PATENT APPLICATION**

(21) Application number: **90102725.0**

(51) Int. Cl.5: **C12P 13/04, C12P 13/08**

(22) Date of filing: **12.02.90**

(30) Priority: **27.02.89 US 316353**

(43) Date of publication of application: **05.09.90 Bulletin 90/36**

(84) Designated Contracting States: **DE FR GB IT**

(71) Applicant: **E.R. SQUIBB & SONS, INC.**
**P.O.Box 4000**
**Princeton New Jersey 08543-4000(US)**

(72) Inventor: **Hanson, Ronald L.**
**54 Whitewood Dr.**
**Morris Plains, NJ 07950(US)**
Inventor: **Szarka, Laszlo J.**
**5 Wellington Rd.**
**East Brunswick, NJ 08816(US)**
Inventor: **Ramesh, Patel N.**
**572 Cabot Hill Rd.**
**Bridgewater, NJ 08807(US)**

(74) Representative: **Josif, Albert et al**
**Baaderstrasse 3**
**D-8000 München 5(DE)**

(54) **Process for transformation of hydroxyketo acids to hydroxyamino acids.**

(57) A novel process for preparation of hydroxyamino acids is disclosed. In this process, a hydroxyketo acid reacts with a nitrogen source when treated with an amino acid dehydrogenase, a transaminase, or microorganisms that produce such enzymes. This process allows production of compounds having the formula

III

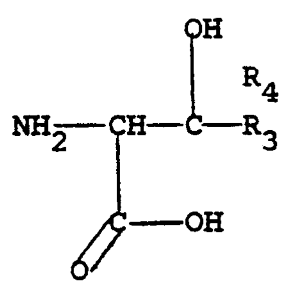

from compounds having the formula

EP 0 385 172 A1

IV

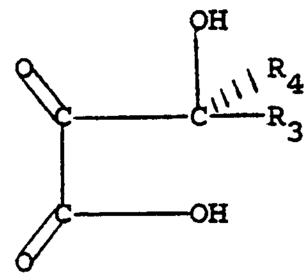

wherein $R_3$ and $R_4$ are each independently hydrogen, alkyl, or aryl. In one embodiment of the invention, the transformation to hydroxyamino acid is combined with NADH regeneration by treatment with a dehydrogenase and its associated substrate.

# PROCESS FOR TRANSFORMATION OF HYDROXYKETO ACIDS TO HYDROXYAMINO ACIDS

This invention relates to preparation of chemical intermediates required for synthesis of antibiotics, and in particular to intermediates for Tigemonam and related compounds.

Tigemonam and its related compounds are O-sulfated, β-lactam hydroxamic acids having antibacterial activity. These compounds can be described by the formula

I

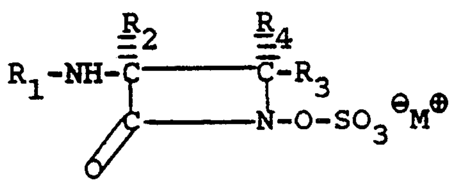

wherein, in formula I and throughout the specification,

$R_1$ is acyl;

$R_2$ is hydrogen or alkoxy;

$R_3$ is hydrogen, aryl, or alkyl;

$R_4$ is hydrogen, aryl, or alkyl; and

$M^+$ is hydrogen or a cation.

Formula I compounds can combat bacterial infections, including respiratory and urinary infections, in mammalian species. Uses of these compounds are disclosed in U.S. Patent Nos. 4,751,220 and 4,638,061.

The β-lactams of formula I can be prepared from the corresponding hydroxamic acid having the formula

II

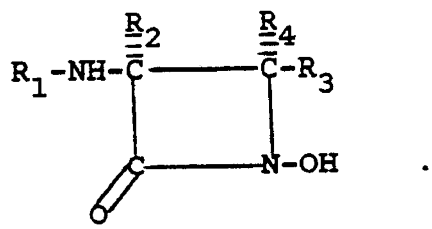

Formula II compounds can be prepared from corresponding hydroxyamino acids having the formula

III

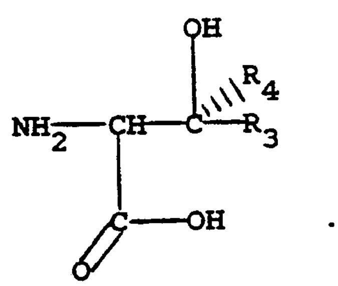

Formula I compounds can be derived from formula II and III compounds as described in patent no. 4,533,660, issued August 6, 1985. Thus, formula III compounds, particularly L-β-hydroxyvaline, are key intermediates required for synthesis of useful formula I compounds. Because formula III compounds are needed to produce the antibacterial agents of formula I, methods of producing formula III compounds are

quite useful.

Existing methods for producing formula III compounds are dependent on chemical synthesis and resolution of racemic mixtures, as indicated in the following references:

1. Belokon et al. General Method of Diastereo and Enantio Selective Synthesis of β-hydroxy-α-Amino Acid by Condensation of Aldehydes and Ketones with Glycine, J. Am. Chem. Soc. 107: 4252-4259 (1985).
2. Slusarchyk et al. β-Lactam Synthesis: Chemospecific Sulfonation and Cyclization of the β-hydroxyvaline Nucleus, Tetrahedron Letters 27: 2789-2792 (1986).
3. Edwards, G. W. and Minthorn, M. L., Jr. Preparation and Properties of the Optical Antipodes of β-Methoxyvaline and β-Hydroxyvaline, Can. J. Biochem. 46:1227-1230.
4. Mix, Hermann. Synthese Aliphatischer 2-Amino-3-Hydroxysäuren und Diastereomerentrennung mit Hilfe Kupferchelaten, Z. Physiol. Chem. 327:41-48 (1961).
5. Schöllkopf, U. et al. Asymmetric Syntheses via Heterocyclic Intermediates XV Enantioselective Synthesis of (R)-(-)-β-hydroxyvaline Using L-valine or (S)-0,0-Dimethyl-α-Methyldopa as Chiral Ancillary Reagents, Synthesis 10: 868-870 (1982).
6. Ohashi J. and Hirada, K. Synthesis, Resolution, and Configuration of β-Hydroxyvaline, Bull. Chem. Soc. Japan 39 (10): 2287-2289 (1966).
7. Ohashi, Y. et al. Chemistry of the Antibiotic YA-56 II. β-hydroxy-L-Valine and 4-Amino-3,6-Dihydroxy-2-Methylhexanoic Acid, Constitutional Amino Acids of the Antibiotic YA-56, Agr. Biol. Chem. 37 (10), 2283-2287 (1973).

These existing methods have significant disadvantages. Synthetic chemical methods for producing β-hydroxyvaline depend upon resolution of racemic D-L-β-hydroxyvaline which gives a yield of L-β-hydroxyvaline of less than 50%; the other half, D-β-hydroxyvaline, is an undesired isomer obtained during the resolution process. In a case where enantiomeric purity of 70% was obtained, the undesired D-β-hydroxyvaline was the predominant product (reference 1).

The present invention differs from chemical synthesis. The biocatalytic synthesis of L-β-hydroxyvaline using enzymes or microbial cells results in up to 100% optical purity and up to 98% yield.

In accordance with the present invention, a novel process for preparing hydroxyamino acids is disclosed. In particular, a novel process for preparing such formula III compounds as L-β-hydroxyvaline is disclosed. This novel process comprises reductive amination or transamination of hydroxyketo acids, particularly compounds having the formula

IV

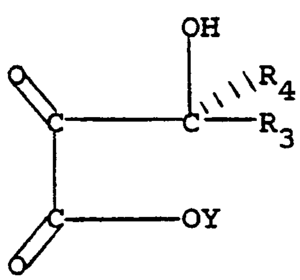

wherein, in formula IV and throughout the specification, Y is alkali metal, hydrogen, or alkyl.

Formula IV compounds are derived by treating corresponding keto acids or their esters first with a halogen and then with an alkali metal hydroxide. An alternative method is Darzens condensation of acetone with alkyldihaloacetates. The α-haloglycidic ester is then hydrolyzed to formula IV with sodium bicarbonate and/or sodium hydroxide.

In this invention, the hydroxyketo acids represented by formula IV are reacted with a nitrogen source, such as an ammonium salt. The reaction takes place in the presence of an amino acid dehydrogenase, a transaminase, or microorganisms that produce such enzymes. In one embodiment of the invention, the reaction is used in conjunction with regeneration of reduced nicotinamide adenine dinucleotide ($NAD^+$ in its oxidized form: NADH in reduced form) by treatment with a dehydrogenase and its associated substrate.

The process of the present invention has several advantages. Optically pure L-β-hydroxyamino acid is obtained in high yield. When whole microbial cells are used, the expensive $NAD^+$ cofactor need not be supplied. When amino acid dehydrogenases are used, the expensive NADH cofactor can be regenerated and reused. Enzymes or microbial cells can be reused for several cycles of synthesis.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred.

The term "halogen" refers to fluorine, chlorine, bromine and iodine.

The term "alkali metal" refers to lithium, sodium, potassium, rubidium, cesium, and francium.

The term "alkali metal hydroxide" refers to LiOH, NaOH, KOH, RbOH, CsOH, and FrOH.

The term "acyl" refers to those groups identified in patent no. 4,533,660, issued August 6, 1985.

The term "aryl" refers to phenyl and substituted phenyl. Exemplary substituted phenyl groups are substituted with 1, 2 or 3 amino ($-NH_2$), alkylamino, dialkylamino, nitro, halogen, hydroxyl, trifluoromethyl, alkyl (of 1 to 4 carbon atoms), alkoxy (of 1 to 4 carbon atoms), alkylthio (of 1 to 4 carbon atoms), alkanoyloxy, carbamoyl, or carboxyl groups.

The term "cation", as used throughout the specification, refers to any positively charged atom or group of atoms. The "$-O-SO_3^{\ominus}M^{\oplus}$ substituent on the nitrogen atom of the β-lactams encompasses all sulfate salts. Pharmaceutically acceptable salts are, of course, preferred, although other salts are also useful in purifying formula compounds or as intermediates for the preparation of pharmaceutically acceptable salts. The cationic portion of the sulfonic acid salts can be obtained from either organic or inorganic bases. Such cationic portion includes, but is not limited to, the following ions: ammonium; substituted ammonium, such as alkylammonium (e.g., tetra-n-butylammonium, referred to hereinafter as tetrabutylammonium); alkali metal, such as lithium, sodium and potassium; alkaline earth metal, such as calcium and magnesium; pyridinium; dicyclohexylammonium; hydrabaminium; benzathinium; N-methyl-D-glucaminium.

The term "substituted ammonium" refers to such compounds as $NH_4+$, ammonium hydroxide, ammonium phosphate and other ammonium salts.

As set forth in formula I, and in the definitions following formula I, $M^{\oplus}$ can be hydrogen provided the $R_1$ group contains a basic function. Such compounds are often referred to in the art as "inner salts" by virtue of a positive and negative charge in the molecule.

In the process of the present invention, the starting material is either a keto acid or ester having the formula

VI(I)

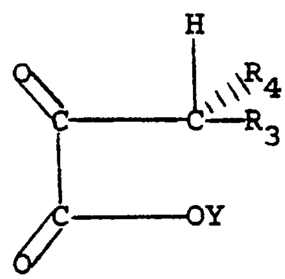

or an α-halo-glycidic ester having the formula

VI(II)

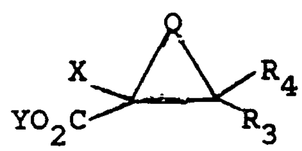 .

Methods of producing formula VI compounds are well known.

The formula VI(I) compound is treated with a halogen, yielding a compound following the formula

V

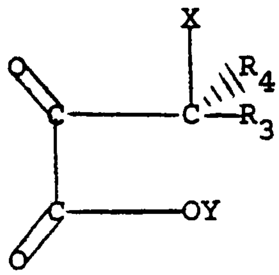

wherein X is halogen. In effect, a halogen replaces a hydrogen in formula VI(I).

The formula V compound is then treated with an alkali or base metal hydroxide (e.g., NaOH), which reacts with the halogen and forms a compound of formula

IV

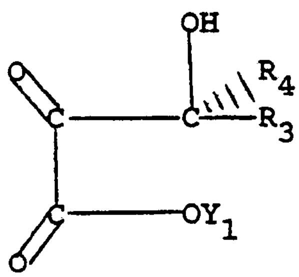

wherein $Y_1$ is an alkali metal atom.

Alternatively, the formula VI(II) compound is treated with alkali metal bicarbonate and/or alkali metal hydroxide to form a compound of formula IV.

The next step in the process is the reductive amination or transamination of the formula IV compound to form the desired corresponding formula III compound. The formula IV compound is treated with a nitrogen source in the presence of either (1) an appropriate micoorganism or (2) NADH and an appropriate enzyme.

Appropriate nitrogen sources include ammonia, ammonium ion, substituted ammonium, ammonium formate, ammonium chloride, ammonium phosphate, and other ammonium and substituted ammonium salts. Microbial cells can be used either in the free state or immobilized on solid support.

Appropriate microorganism genuses include:

Bacillus
Corynebacteria
Alcaligenes
Clostridium
Micrococcus
Pseudomonas
Escherichia
Candida
Aerobacter
Flavobacterium
Rhizobium
Acetobacter
Proteins
Sarcina
Mucor
Rhizopus
Saccharomyces
Torulopsis

Salmonella
Aspergillus
Penicillium
Rhodotorula
and the like. Specific microorganism species suitable for this process include:
Bacillus megaterium
Bacillus sphaericus
Bacillus subtilis
Bacillus cereus
Bacillus pumilus
Bacillus licheniformis
Bacillus thuringiensis
Bacillus brevis
and the like.

Bacillus sphaericus is preferred for this process. Although B. sphaericus in the past has been found inactive toward serine and threonine in the reverse reaction (oxidative deamination), it has given yields as high as 100% for synthesis of L-$\beta$-hydroxyvaline in this process. A particularly effective and preferred strain is B. sphaericus A.T.C.C. 4525 (American Type Culture Collection, 12301 Parklawn Drive, Rockville, MD 20852).

Appropriate microorganisms can be grown in conventional media (for example, with glucose as a carbon source and yeast extracts and tryptone as nitrogen and amino acid sources) to induce the amino acid dehydrogenases or transaminases required for the transformation to hydroxyamino acid.

Microorganisms used as whole cells for this process also provide NADH. Thus, it is unnecessary to supply NADH separately when intact microorganisms are used.

when intact microorganisms are not used in the process, it is useful to add an NADH regeneration function. In this modified process, a hydroxyketo acid is placed in the presence of an $NAD^+$ source, a nitrogen source, an amino acid dehydrogenase, a second dehydrogenase, and a substrate associated with the second dehydrogenase. The substrate loses a hydrogen atom to the $NAD^+$, yielding NADH; the amino acid dehydrogenase allows the nitrogen source and NADH to react with the hydroxyketo acid, yielding a hydroxyamino acid and $NAD^+$ to repeat the process. For example, leucine dehydrogenase may be placed in the presence of $NAD^+$, ammonium salt, a formula IV compound, formate dehydrogenase, and formate. The formate loses a hydrogen atom to $NAD^+$, yielding NADH; the NADH and leucine dehydrogenase allow the formula IV compound to react with the ammonium salt, yielding a formula III compound with $NAD^+$. (Glucose and primary and secondary alcohols are also effective substrates when used with appropriate dehydrogenases.)

Leucine dehydrogenase is the preferred amino acid dehydrogenase.

For the NADH generation, preferred dehydrogenases include:
glucose dehydrogenase
formate dehydrogenase
primary alcohol dehydrogenase
secondary alcohol dehydrogenase
glycerol dehydrogenase
and the like.

Preferred transaminases include branched chain amino acid transaminases, especially leucine, isoleucine, and valine transaminases.

Although NADH is preferred, NADH derivatives may be used as the $NAD^+$ source instead of NADH. Appropriate $NAD^+$ sources include dextran-NAD, polyethylene glycol-$N^6$-(2-aminoethyl)-NADH, and the like.

The present invention will now be described by the following examples. Temperatures are given in degrees Celsius, unless stated otherwise. Other examples are possible, as will be understood by those skilled in the art. These examples are illustrative rather than limiting, so that the scope of this invention is limited only by the claims appended hereto.

## Example 1

Bacillus sphaericus A.T.C.C. 4525 was grown at 30° in a 250 L fermentor on a medium containing per L: 20 g yeast extract, 10 g glucose and 2 g $KH_2PO_4$, pH 7. Cells were harvested when absorbance at 650

nm was 2.8 and stored frozen until used. 5 g of frozen cells were suspended in 20 ml of 0.1 M potassium phosphate, pH 7, containing 0.01% mercaptoethanol, disrupted by sonication for 5 minutes, heated 20 minutes at 60°, and centrifuged 10 minutes at 27000 X g. The supernatant contained 24 units/ml and 12 units/mg protein of leucine dehydrogenase. (1 unit converts l µmole 2-ketoisovalerate to valine per minute).

To ice-cold ethyl-2-keto-β-bromoisovalerate (446 mg, 2 mmoles) was added 5 N sodium hydroxide (0.84 ml, 4.2 mmoles) and the volume was brought to 4 ml. After stirring for 1 hour at room temperature, a 0.5 M solution of α-keto-β-hydroxyisovalerate was obtained. 1 ml reactions were set up containing either:

1. 1 M ammonium formate, 0.1 M α-keto-β-hydroxyisovalerate, 0.01% mercaptoethanol, 5 units/ml formate dehydrogenase from Candida boidinii and 6 units/ml leucine dehydrogenase, or

2. 1 M glucose, 1 M ammonium chloride, 0.1 M α-keto-β-hydroxyisovalerate, 0.01% mercaptoethanol, 5 units/ml glucose dehydrogenase from Bacillus megaterium and 6 units/ml leucine dehydrogenase. Each reaction also contained 1 mM pyridine nucleotide cofactor as indicated in Table 1. Reaction mixtures were incubated for 22 hours at 30°. L-β-hydroxyvaline concentration was determined by HPLC analysis with a Bakerbond Chiralpak WH column. Yields with the three cofactors and two recycling systems are given in Table 1.

## Example 2

Bacillus sphaericus A.T.C.C. 4525 was grown at 30°C in shake flasks to absorbance of 2.2 at 600 nm on medium containing per L: 1 g yeast extract, 17 g "tryptone", 3 g "soytone", 5 g sodium chloride, 2.5 g glucose and 2.5 g $K_2HPO_4$ adusted to pH 7 with hydrochloric acid. Leucine dehydrogenase was partially purified by heat treatment as described in Example 1.

2-Chloro-3,3-dimethyloxiranecarboxyclic acid, methyl ester (658 mg, 4 mmoles), was added to 7 ml water containing sodium bicarbonate (370 mg, 4.4 mmoles). The mixture was shaken overnight at room temperature. 5 N sodium hydroxide (0.8 ml, 4 mmoles) was added and shaking was continued for 8 hours to give a clear solution. Glucose (2.88 g, 16 mmoles) and ammonium chloride (171.2 mg, 3.2 mmoles) were added, and pH was adjusted to 8.5 with sodium hydroxide. β-Mercaptoethanol (1.6 µl), NAD (4.2 mg, 6 mmoles), leucine dehydrogenase (44 units) and glucose dehydrogenase from B. megaterium (29 units) were added and volume was adjusted to 16 ml. The reaction was carried out on a pH stat at 30° with pH 8.5 maintained by addition of 3 M ammonium hydroxide. After 63 hours, conversion to L-β- hydroxyvaline was 98%, based on the starting concentration of 2-chloro-3,3-dimethyloxiranecarboyxlic acid, methyl ester.

## Example 3

2-Chloro-3,3-dimethyloxiranecarboxylic acid, isopropyl ester (770 mg, 4 mmoles), was treated with sodium bicarbonate and sodium hydroxide as described in Example 2. The enzyme reaction was carried out with a pH stat as described in Example 2. After 64 hours, conversion to L-β-hydroxyvaline was 71%, based on the initial concentration of the ester.

## Example 4

2-Chloro-3,3-dimethyloxiranecarboxylic acid, 1,1-dimethylethyl ester (826 mg, 4 mmoles), was added to 8 ml water containing the sodium bicarbonate (370 mg, 4.4 mmoles) and shaken overnight at room temperature. 5 N sodium hydroxide (1.28 ml, 6.4 mmoles) was added and shaking was continued for 54 hours. The enzyme reaction was carried out with a pH stat as described in Example 2. After 66 hours, conversion to L-β-hydroxyvaline was 83%.

## Example 5

Bacillus cereus A.T.C.C. 14579 was grown at 30° in shake flasks on the medium described in Example 2 to an absorbance of 2.9 at 600 nm. Cells were harvested by centrifugation and washed with 0.1 M

potassium phosphate, pH 7. 5 N sodium hydroxide (0.672 ml, 3.36 mmoles) and 7 ml water were added to ice-cold ethyl α-keto-β-bromoisovalerate (357 mg, 1.6 mmoles). The mixture was shaken for 1 hour, then glucose (2.88 g, 16 mmoles) and ammonium chloride (171.2 mg, 3.2 mmoles) were added and pH was adjusted to 8. B. cereus intact cells (1.6 g wet weight) were added and volume was brought to 16 ml. The reaction was carried out with a pH stat at 30° with pH maintained at 8 by addition of 3 M ammonium hydroxide. After 24 hours, conversion to L-β-hydroxyvaline was 50%, based on starting concentration of ethyl-α-keto-β-bromoisovalerate.

## Example 6

α-Keto-β-bromoisovaleric acid (390 mg, 2 mmoles) was cooled on ice and dissolved in 2 N sodium hydroxide (2.10 ml, 4.2 mmoles). Ammonium formate (504.5 mg, 8 mmoles) was added and the solution was brought to 4 ml, pH 8, with sodium hydroxide (5.3 mg, 8 mmoles), leucine dehydrogenase from Bacillus species (Sigma Chemical Company, 16 units) and formate dehydrogenase from Candida boidinii - (40 units) were added. After 24 hours at 30°, conversion to L-β-hydroxyvaline was 82%, based on the starting concentration of α-keto-β-bromoisovaleric acid.

Table 1

**Effect of Pyridine Nucleotide Derivatives on Synthesis of L-β-Hydroxyvaline**

| Pyridine Nucleotide | L-β-Hydroxyvaline, mM | |
|---|---|---|
| | Formate Dehydrogenase | Glucose Dehydrogenase |
| 1 mM PEG-NADH (1) | 82.1 | 10.1 |
| 1 mM Dextran-NAD (2) | 34.1 | 79.9 |
| 1 mM NAD | 81.8 | 77.3 |

1. Polyethyleneglycol (MR 20000)-N6-(2-aminoethyl)-NADH
2. Attached through C8 to soluble dextran, D4133, with a 6 carbon spacer

## Claims

1. A process for transforming a hydroxyketo acid to a hydroxyamino acid by reductive amination or transamination, which comprises:

(a) mixing a nitrogen source and a hydroxyketo acid; and

(b) treating the hydroxyketo acid with an amino acid dehydrogenase, a transaminase, an amino acid dehydrogenase-producing microorganism, or a transaminase-producing microorganism.

2. A process for preparing a compound having the formula

III

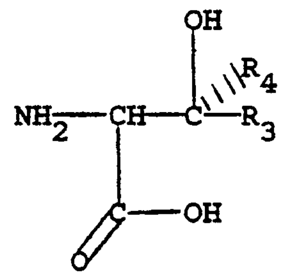

wherein $R_3$ and $R_4$ are each independently hydrogen, aryl, or alkyl, and wherein the process comprises:

(a) mixing a nitrogen source and a substrate having the formula

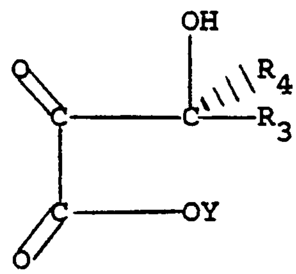

wherein $R_3$ and $R_4$ are each independently hydrogen or alkyl and Y is hydrogen, alkali metal, or alkyl; and

(b) treating the substrate with an amino acid dehydrogenase, a transaminase, an amino acid dehydrogenase-producing microorganism, or a transaminase-producing microorganism.

3. The process of Claim 1, further comprising treating the hydroxyketo acid with a nicotinamide adenine dinucleotide source.

4. The process of Claim 2, further comprising treating the substrate with a nicotinamide adenine dinucleotide source.

5. The process of Claim 3, wherein the nicotinamide adenine dinucleotide source is selected from a group consisting of:
NADH,
dextran-NAD,
polyethylene glycol-$N^6$-(2-aminoethyl)-NADH.

6. The process of Claim 4, wherein the nicotinamide adenine dinucleotide source is selected from a group consisting of:
NADH,
dextran-NAD,
polyethylene glycol-$N^6$-(2-aminoethyl)-NADH.

7. The process of Claim 1, wherein the amino acid dehydrogenase is leucine dehydrogenase.

8. The process of Claim 2, wherein the amino acid dehydrogenase is leucine dehydrogenase.

9. The process of Claim 1, wherein the transaminase is a branched chain transaminase.

10. The process of Claim 2, wherein the transaminase is a branched chain transaminase.

11. The process of Claim 9, wherein the branched chain transaminase is selected from a group consisting of leucine transaminase, isoleucine transaminase, and valine transaminase.

12. The process of Claim 10, wherein the branched chain transaminase is selected from a group consisting of leucine transaminase, isoleucine transaminase, and valine transaminase.

13. The process of Claim 1, wherein the microorganism is selected from a group consisting of: Bacillus
Corynebacteria
Alcaligenes
Clostridium
Micrococcus
Pseudomonas

Escherichia
Aerobacter
Flavobacterium
Rhizobium
Salmonella
Acetobacter
Proteus
Sarcina
Mucor
Rhizopus
Saacharomyces
Torulopsis
Aspergillus
Penicillium
Rhodotorula
Candida.

14. The process of Claim 2, wherein the microorganism is selected from a group consisting of: Bacillus
Corynebacteria
Alcaligenes
Clostridium
Micrococcus
Pseudomonas
Escherichia
Aerobacter
Flavobacterium
Rhizobium
Candida
Acetobacter
Proteus
Sarcina
Mucor
Rhizopus
Saacharomyces
Torulopsis
Aspergillus
Penicillium
Rhodotorula
Salmonella.

15. The process of Claim 1, wherein the microorganism comprises Bacillus sphaericus.

16. The process of Claim 1, further comprising placing the nitrogen source and the hydroxyketo acid in presence of a nicotinamide adenine dinucleotide source, a dehydrogenase, and a substrate associated with the dehydrogenase, so that oxidized nicotinamide adenine dinucleotide may be reduced, used to transform the hydroxyketo acid to a hydroxyamino acid, and returned to its oxidized form in a continuing process.

17. The process of Claim 2, further comprising placing the nitrogen source and the formula IV compound in presence of a nicotinamide adenine dinucleotide source, a dehydrogenase, and a substrate associated with the dehydrogenase, so that oxidized nicotinamide adenine dinucleotide may be reduced, used to transform the formula IV compound to a formula III compound, and returned to its oxidized form in a continuing process.

18. The process of Claim 21, wherein the substrate is selected from a group consisting of formate, glucose, glycerol and primary and secondary alcohols.

19. The process of Claim 22, wherein the substrate is selected from a group consisting of formate, glucose, glycerol and primary and secondary alcohols.

20. The process of Claim 16, wherein the nicotinamide adenine dinucleotide source is selected from a group consisting of:
NADH,
dextran-NAD,
polyethylene glycol-$N^6$-(2-aminoethyl)-NADH.

21. The process of Claim 17, wherein the nicotinamide adenine dinucleotide source is selected from a

group consisting of:
NADH,
dextran-NAD,
polyethylene glycol-$N^6$-(2-aminoethyl)-NADH.

22. The process of Claim 1, wherein the nitrogen source is selected from a group consisting of ammonia, ammonium ion, substituted ammonium, and ammonium and substituted ammonium salts.

23. The process of Claim 2, wherein the nitrogen source is selected from a group consisting of ammonia, ammonium ion, substituted ammonium, and ammonium and substituted ammonium salts.

24. The process of Claim 2, wherein the microorganism comprises Bacillus sphaericus.

25. The process of Claim 2, wherein the microorganism comprises Bacillus sphaericus A.T.C.C. 4525.

26. The process of Claim 2, wherein the microorganism comprises Bacillus sphaericus A.T.C.C. 4525.

27. The process of Claim 1, wherein the microorganism is selected from a group consisting of:
Bacillus sphaericus,
Bacillus megaterium,
Bacillus subtilis,
Bacillus cereus,
Bacillus pumilus,
Bacillus licheniformis,
Bacillus thuringiensis,
Bacillus brevis.

28. The process of Claim 2, wherein the microorganism is selected from a group consisting of:
Bacillus sphaericus,
Bacillus megaterium,
Bacillus subtilis,
Bacillus cereus,
Bacillus pumilus,
Bacillus licheniformis,
Bacillus thuringiensis,
Bacillus brevis.

European Patent Office

# EUROPEAN SEARCH REPORT

Application Number

EP 90 10 2725

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 206 904 (INSTITUT FRANCAIS DU PETROLE)<br>* Whole document *<br>--- | 1-8,13-14,16-23,27-28 | C 12 P 13/04<br>C 12 P 13/08 |
| X | EP-A-0 140 503 (GENENTECH, INC.)<br>* Abstract; claims *<br>--- | 1-6,13-14,18-19,22-23 | |
| X | EP-A-0 023 346 (DEGUSSA AG)<br>* Abstract; claims *<br>--- | 1-6,16-23 | |
| A | DE-A-3 427 495 (W.R. GRACE & CO.)<br>* Claims 1,10 *<br>--- | 2 | |
| X,P | FR-A-2 628 751 (INSTITUT FRANCAIS DU PETROLE)<br>* Abstract; example 1; claims *<br>----- | 1-8,13-14,16-23,27-28 | |
| | | | TECHNICAL FIELDS SEARCHED (Int. Cl.5) |
| | | | C 12 P |

The present search report has been drawn up for all claims

| Place of search | Date of completion of the search | Examiner |
|---|---|---|
| THE HAGUE | 17-05-1990 | ALVAREZ Y ALVAREZ C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)